# EUROPÄISCHE PATENTSCHRIFT

(11) **EP 2 976 280 B1**
(45) Veröffentlichungstag und Bekanntmachung des Hinweises auf die Patenterteilung: **10.05.2017**
(21) Anmeldenummer: 14710882.3
(22) Anmeldetag: 17.03.2014
(51) Int. Cl.: B65H 45/12, A61F 13/00, A61M 5/00

(54) **VERFAHREN UND VORRICHTUNG ZUM FALZEN EINES DECKMATERIALS AN EINEM MEDIZINISCHEN SENSOR**
METHOD AND DEVICE FOR FOLDING A COVER MATERIAL ON A MEDICAL SENSOR
PROCÉDÉ ET DISPOSITIF SERVANT À PLIER UN MATÉRIAU DE RECOUVREMENT AU NIVEAU D'UN DISPOSITIF MÉDICAL DE DÉTECTION

(30) Priorität: 19.03.2013 DE 102013004672
(43) Veröffentlichungstag der Anmeldung: 27.01.2016
(73) Patentinhaber: Fresenius Medical Care Deutschland GmbH, 61352 Bad Homburg (DE)
(72) Erfinder: HEPPE, John, 66606 St. Wendel (DE); RULLOF, Roland, 66822 Lebach (DE)
(74) Vertreter: Oppermann, Frank
(86) Internationale Anmeldenummer: PCT/EP2014/055346
(87) Internationale Veröffentlichungsnummer: WO 2014/147040

(56) Entgegenhaltungen:
- EP-A1- 2 325 122
- WO-A1-2008/032623
- WO-A1-2011/116943
- DE-A1- 3 935 003
- GB-A- 2 297 260
- US-A- 5 162 040

## Beschreibung

Die Erfindung betrifft ein Verfahren und eine Vorrichtung zum Falzen eines Deckmaterials an einem medizinischen Sensor, der ein insbesondere an der Haut eines Patienten zu befestigendes Trägermaterial aufweist, das an der dem Patienten zugewandten Seite mit einer Adhäsionsschicht versehen ist, auf die das Deckmaterial aufgebracht ist, wobei ein Abschnitt des Deckmaterials, der einem von der Adhäsionsschicht freien Abschnitt des Trägermaterials gegenüberliegt, eine Lasche bildet.

Auf dem Gebiet der Medizintechnik sind verschiedene Vorrichtungen bekannt, mit denen über eine Schlauchleitung Patienten Flüssigkeit entnommen oder Flüssigkeiten dem Patienten zugeführt werden können. Dabei erfolgt der Zugang zu dem Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patient sicherzustellen. Daher ist es erforderlich, den Patientenzugang zu überwachen. Einen ordnungsgemäßen Patientenzugang setzen insbesondere die extrakorporalen Blutbehandlungsvorrichtungen voraus, die über einen extrakorporalen Blutkreislauf verfügen.

Zur Überwachung eines Gefäßzugangs bei extrakorporalen Blutbehandlungsvorrichtungen sind Vorrichtungen bekannt, die über einen elektrischen Feuchtigkeitssensor verfügen, um an der Punktionsstelle austretendes Blut detektieren zu können. Die bekannten Feuchtigkeitssensoren sind als Pad ausgebildet, das auf die Punktionsstelle aufgelegt werden.

Aus der WO 2011/116943 A1 ist ein gewebter Feuchtigkeitssensor bekannt, der über eine Anschlusslasche verfügt, an der die elektrischen Anschlusskontakte angeordnet sind. Der Sensor weist ein textiles Flächengebilde auf, das auf die Haut des Patienten aufgelegt wird. Das textile Flächengebilde des Sensors ist mit einer Klebe- oder Adhäsionsschicht versehen, die von einem abziehbaren Deckmaterial abgedeckt wird. Die Klebe- oder Adhäsionsschicht ist derart ausgebildet, dass der Sensor nach dem Abziehen des Deckmaterials an der Haut des Patienten befestigt werden kann.

Die medizinischen Sensoren sollten sich in großen Stückzahlen kostengünstig herstellen lassen. Für eine einfache Handhabung der medizinischen Sensoren sollte sich das Deckmaterial leicht von dem Trägermaterial abziehen lassen.

Der Erfindung liegt die Aufgabe zugrunde, ein Verfahren bereitzustellen, mit dem die Herstellung von medizinischen Sensoren, die sich leicht handhaben lassen, in großen Stückzahlen vereinfacht wird. Darüber hinaus ist eine Aufgabe der Erfindung, eine Vorrichtung zu schaffen, mit der die Herstellung von leicht zu handhabenden medizinischen Sensoren in großen Stückzahlen vereinfacht wird. Insbesondere liegt der Erfindung die Aufgabe zugrunde, ein Verfahren und eine Vorrichtung zum Falzen eines Deckmaterials an einem medizinischen Sensor bereitzustellen.

Die Lösung dieser Aufgaben erfolgt erfindungsgemäß mit den Merkmalen der unabhängigen Ansprüche. Vorteilhafte Ausführungsformen der Erfindung sind Gegenstand der abhängigen Ansprüche.

Das erfindungsgemäße Verfahren und die erfindungsgemäße Vorrichtung erlauben die Herstellung von medizinischen Sensoren, bei denen ein Abschnitt des Deckmaterials eine abstehende Lasche bildet, die sich leicht greifen lässt.

Die Lasche wird dadurch geschaffen, dass das Trägermaterial an einer Seite in einem ersten Abschnitt eine Adhäsions- oder Klebeschicht und in einem zweiten Abschnitt keine Adhäsions- oder Klebeschicht aufweist, so dass das Deckmaterial in dem zweiten, von der Adhäsions- oder Klebeschicht freien Abschnitt nicht mit dem Trägermaterial verklebt ist. Das erfindungsgemäße Verfahren und die erfindungsgemäße Vorrichtung sehen vor, die Lasche umzulegen und mit einer Falzkante zu versehen.

Nach dem Falzen des Deckmaterials kann die von dem Trägermaterial abstehende Lasche auch mit Schutzhandschuhen leicht gegriffen werden. Dies ist insbesondere bei medizinischen Sensoren von Vorteil, die unter sterilen Bedingungen mit Schutzhandschuhen gehandhabt werden müssen, insbesondere bei Feuchtigkeitssensoren zur Überwachung eines Gefäßzugangs.

Unter Adhäsions- oder Klebeschicht wird nachfolgend jede Schicht verstanden, die einerseits eine ausreichende Verbindung zwischen Träger- und Deckmaterial schafft und andererseits ein Abziehen des Deckmaterials von dem Trägermaterial erlaubt. Darüber hinaus sollte sich mit der Adhäsions- oder Klebeschicht auch eine ausreichend haftende Verbindung zwischen dem Trägermaterial und der Haut des Patienten herstellen lassen.

Das Herstellungsverfahren erlaubt die vollflächige Aufbringung des Deckmaterials auf das Trägermaterial, ohne dass überlappende Abschnitte erforderlich sind, um die Lasche besser greifen zu können. Da die Lasche nicht übersteht, lässt sich der Sensor leichter aus dem Verbund von Trägermaterial und Deckmaterial zuschneiden oder herausstanzen als wenn überlappende Bereiche vorhanden wären, wodurch die Herstellungskosten der Sensoren verringert werden. Zum Separieren der Sensoren aus einer Materialbahn ist nur ein Schnitt- oder Stanzvorgang erforderlich.

Das erfindungsgemäße Verfahren und die erfindungsgemäße Vorrichtung beruhen auf der Verwendung eines Werkzeugs zum Falzen des Deckmaterials, das mindestens zwei Anlageflächen aufweist, die sich in einer parallel zu der Ebene des Verbundes von Trägermaterial und Deckmaterial verlaufenden Kante schneiden. Das Werkzeug wird auf einer schräg zu der Ebene des Verbundes von Trägermaterial und Deckmaterial verlaufenden Achse derart bewegt, dass die Lasche von dem Werkzeug erfasst und mit dem Werkzeug umgebogen wird. Wenn das Werkzeug auf die Lasche zubewegt wird, ist die erste Anlagefläche des Werkzeugs der Lasche zugewandt und die zweite Anlagefläche der Lasche abgewandt. Beim Vorschub des Werkzeugs wird die Lasche von der ersten Anlagefläche erfasst. Die umgelegte Lache wird nach dem Umlegen von der zweiten Anlagefläche gehalten. Zur Herstellung einer Falzkante wird Druck auf den gebogenen Abschnitt des Trägermaterials aufgebracht, so dass die Lasche auch nach Entfernen des Werkzeugs in der umgebogenen Stellung verbleibt.

In der Praxis hat sich gezeigt, dass bei einer maschinellen Kaschierung des Deckmaterials auch in dem von der Adhäsions- oder Klebeschicht freien Abschnitt des Trägermaterials aufgrund von Adhäsionskräften das von der Adhäsions- oder Klebeschicht freie Trägermaterial und die Lasche aneinander haften können. In diesem Fall kann die Lasche von dem Werkzeug nicht sicher erfasst werden. Daher sieht eine bevorzugte Ausführungsform eine Trennung des von der Adhäsions- oder Klebeschicht freien Abschnitts des Trägermaterials von der Lasche vor, bevor das Werkzeug zum Einsatz kommt.

Soweit die Lasche sicher erfasst und umgebogen wird, können die Positionierung des Werkzeugs und die Ausrichtung der Anlageflächen verändert werden. Eine optimale Positionierung und Ausrichtung ist aber gegeben, wenn die erste Anlagefläche im Wesentlichen orthogonal zu der Achse verläuft, auf der das Werkzeug bewegt wird, und die zweite Anlagefläche in einer Ebene liegt, die im Wesentlichen parallel zu der Ebene des Verbundes von Trägermaterial und Deckmaterial verläuft. Dadurch wird erreicht, dass die Lasche während des Vorschubs des Werkszeugs sicher erfasst und gleichmäßig umgebogen wird.

Für die Herstellung einer definierten Falzkante ist eine ausreichende Abstützung des Trägermaterials erforderlich, wenn Druck auf den gebogenen Abschnitt des Deckmaterials aufgebracht wird. Eine bevorzugte Ausführungsform sieht daher eine dritte Anlagefläche an dem Werkzeug vor, die der zweiten Anlagefläche gegenüberliegt. Beim Aufbringen von Druck auf den gebogenen Abschnitt des Deckmaterials wird der gebogene Abschnitt des Deckmaterials gegen die dritte Anlagefläche gedrückt. Die zweite und dritte Anlagefläche sind dann optimal zueinander ausgerichtet, wenn sie im Wesentlichen parallel zueinander verlaufen.

Die dritte Anlagefläche ist vorzugsweise an dem vorzugsweise einstückigen Werkzeug vorgesehen. Es ist aber auch möglich, dass die Anlagefläche zum Abstützen des Trägermaterials von einem Teil gebildet wird, der nicht einstückiger Bestandteil des Werkzeugs ist.

Die Länge der zweiten Anlagefläche sollte so bemessen sein, dass die umgebogene Lasche an der dem Trägermaterial gegenüberliegenden Seite über ihre im Wesentlichen gesamte Länge erfasst wird, während die Länge der dritten Anlagefläche so bemessen sein sollte, dass nur der Abschnitt des umgebogenen Deckmaterials im Bereich der Biegung erfasst wird. Daher springt die dritte Anlagefläche gegenüber der ersten Anlagefläche zurück. Die Länge der zweiten Anlagefläche sollte also etwa der Länge der umgebogenen Lasche und die Länge der dritten Anlagefläche etwa der Länge des Abschnitts des umgelegten Deckmaterials im Bereich der Biegung entsprechen.

Die zweite und dritte Anlagefläche bilden bei einer weiteren bevorzugten Ausführungsform die seitlichen Begrenzungen einer Nut in dem Werkzeug, so dass der Nutgrund einen Anschlag für den umgebogenen Abschnitt des Deckmaterials bildet, um die umgelegte Lasche exakt in die Position bringen zu können, an der die Falzkante liegen soll. Die exakte Positionierung erfolgt vorteilhafterweise dadurch, dass der umgebogene Abschnitt des Abdeckmaterials gegen den Nutgrund gedrückt wird, bevor der umgebogene Abschnitt gegen die dritte Anlagefläche gedrückt wird.

Eine bevorzugte Ausführungsform sieht zur exakten Positionierung ein Positionierungselement vor, das in einer zu der Ebene des Verbundes von Trägermaterial und Deckmaterial parallelen Ebene in die Nut vorgeschoben wird, so dass der umgebogene Abschnitt des Deckmaterials gegen den Nutgrund gedrückt wird.

Zum Ausgleich von Bauteiltoleranzen wird der umgebogene Abschnitt des Deckmaterials bei einer weiteren bevorzugten Ausführungsform um eine vorgegebene Wegstrecke wieder zurückgezogen, bevor der umgebogene Abschnitt des Deckmaterials gegen die dritte Anlagefläche gedrückt wird. Dadurch wird sichergestellt, dass Druck auf den umgebogenen Abschnitt in einem genau definierten Bereich ausgeübt wird.

Der Verbund von Trägermaterial und Deckmaterial wird vorzugsweise auf eine Auflagefläche derart aufgelegt, dass sich die Lasche über die Auflagefläche hinaus erstreckt, so dass die Lasche für das Werkzeug frei zugänglich ist.

Zum Trennen des von der Adhäsions- oder Klebeschicht freien Abschnitts des Trägermaterials von der Lasche ist vorzugsweise ein Greifelement vorgesehen, das an der dem Deckmaterial abgewandten Seite des Trägermaterials angreift. Zum Abheben des Trägermaterials von der Lasche wird das Greifelement um eine parallel zu der Ebene des Verbundes von Trägermaterial und Deckmaterial verlaufende Achse verschwenkt. In der Praxis kann es von Vorteil sein, den betreffenden Abschnitt des Trägermaterials mehrmals mit dem Greifelement zu erfassen und umzubiegen. Während der Schwenkbewegung des Greifelements wird die Lasche an der Seite, die dem Trägermaterial abgewandt ist, vorzugsweise von einem Haltelement gehalten.

Die erfindungsgemäße Vorrichtung zum Falzen eines Deckmaterials verfügt über eine Halteeinrichtung, die eine Auflagefläche zum Auflegen des Sensors aufweist, und das erfindungsgemäße Werkzeug sowie ein Andruckelement zur Herstellung der Falzkante.

Die einzelnen Komponenten der erfindungsgemäßen Vorrichtung können mit den bekannten Linearführungen bewegt werden. Der Antrieb der Komponenten kann mit den bekannten Antriebseinheiten erfolgen, beispielsweise mit pneumatischen und/oder hydraulischen und/oder elektrischen Antrieben. Die Ansteuerung der einzelnen Komponenten der erfindungsgemäßen Vorrichtung zur Durchführung der einzelnen Schnitte des erfindungsgemäßen Verfahrens kann mit einer Steuereinheit vollautomatisch erfolgen. Die erfindungsgemäße Vorrichtung kann auch über eine automatische Beschickungseinrichtung zum Einlegen und Entnehmen der Sensoren (Pads) verfügen, die von der Steuereinheit vollautomatisch gesteuert wird.

Im Folgenden wird ein Ausführungsbeispiel der Erfindung unter Bezugnahme auf die Figuren näher erläutert.

Es zeigen:
Fig, 1 ein Ausführungsbeispiel eines medizinischen Sensors,

Eine bevorzugte Ausführungsform sieht vor, dass die erste Anlagefläche im Wesentlichen orthogonal zu der Achse verläuft, auf der das Werkzeug bewegt wird.

Eine weitere bevorzugte Ausführungsform sieht vor, dass die zweite Anlagefläche in einer Ebene liegt, die im Wesentlichen parallel zu der Ebene des Verbundes von Trägermaterial und Deckmaterial verläuft.

Eine weitere bevorzugte Ausführungsform sieht vor, dass das Werkzeug eine dritte Anlagefläche aufweist, die der zweiten Anlagefläche im Abstand gegenüberliegt, wobei der gebogene Abschnitt des umgelegten Deckmaterials gegen die dritte Anlagefläche gedrückt wird.

Eine weitere bevorzugte Ausführungsform sieht vor, dass die zweite und dritte Anlagefläche im Wesentlichen parallel zueinander verlaufen.

Eine weitere bevorzugte Ausführungsform sieht vor, dass sich die zweite Anlagefläche über die dritte Anlagefläche hinaus erstreckt.

Eine weitere bevorzugte Ausführungsform sieht vor, dass die zweite und dritte Anlagefläche die seitlichen Begrenzungen einer Nut in dem Werkzeug bilden, wobei der umgebogene Abschnitt des Deckmaterials gegen den Nutgrund gedrückt wird, bevor der umgebogene Abschnitt gegen die dritte Anlagefläche gedrückt wird.

Eine weitere bevorzugte Ausführungsform sieht vor, dass der umgebogene Abschnitt des Deckmaterials gegen den Nutgrund mit einem Positionierungselement gedrückt wird, das in einer zu der Ebene des Verbundes von Trägermaterial und Dechmaterial parallelen Ebene in die Nut vorgeschoben wird.

Eine weitere bevorzugte Ausführungsform sieht vor, dass der umgebogene Abschnitt des Deckmaterials um eine vorgegebene Wegstrecke zurückgezogen wird, bevor der umgebogene Abschnitt des Deckmaterials gegen die dritte Anlagefläche gedrückt wird.

Eine weitere bevorzugte Ausführungsform sieht vor, dass während der Schwenkbewegung des Greifelements die Lasche an der Seite, die dem Trägermaterial abgewandt ist, von einem Halteelement gehalten wird.

Eine weitere bevorzugte Ausführungsform sieht vor, dass das Werkzeug (20) eine dritte Anlagefläche aufweist, die der zweiten Anlagefläche im Abstand gegenüberliegt.

Eine weitere bevorzugte Ausführungsform sieht vor, dass die zweite und dritte Anlagefläche die seitlichen Begrenzungen einer Nut in dem Werkzeug bilden.

Eine weitere bevorzugte Ausführungsform sieht vor, dass die Achse, auf der das Positionierungselement verschiebbar geführt ist, parallel zu der Achse verläuft, auf die Halteeinrichtung verschiebbar geführt ist.

Eine weitere bevorzugte Ausführungsform sieht vor, dass das Halteelement (30) an einem Schwenkarm angeordnet ist.

Im Folgenden wird ein Ausführungsbeispiel der Erfindung unter Bezugnahme auf die Figuren näher erläutert.

Es zeigen:
- Fig. 1: ein Ausführungsbeispiel eines medizinischen Sensors,
- Fig. 2: die wesentlichen Verfahrensschritte zur Herstellung des Feuchtigkeitssensors in stark vereinfachter schematischer Darstellung,
- Fig. 3: die erfindungsgemäße Vorrichtung in perspektivischer Darstellung,
- Fig. 4: die erfindungsgemäße Vorrichtung in der Draufsicht,
- Fig. 5A: das Werkzeug zum Falzen und das Halte- und Greifelement der erfindungsgemäßen Vorrichtung in stark vereinfachter schematischer Darstellung, wobei sich das Werkzeug in der Ausgangsposition befindet,
- Fig. 5B: der Schritt des Trennens von Deck- und Trägermaterial mit dem Halte- und Greifelement,
- Fig. 5C: das Werkzeug zum Falzen in der Ausgangsposition, wobei das Halteelement zurückgezogen und das Greifelement nicht dargestellt ist,
- Fig. 5D: das Werkzeug in der die Lasche erfassenden Position,
- Fig. 5E: das Werkzeug in der Position, in der die Lasche umgelegt ist,
- Fig. 5F: der Schritt des Positionierens der Lasche mit dem Po sitionierung selement,
- Fig. 5G: der Sensor in der zurückgezogenen Position und
- Fig. 5H: eine vergrößerte Ansicht des Werkzeugs mit dem Andruckelement in teilweise geschnittener Darstellung.

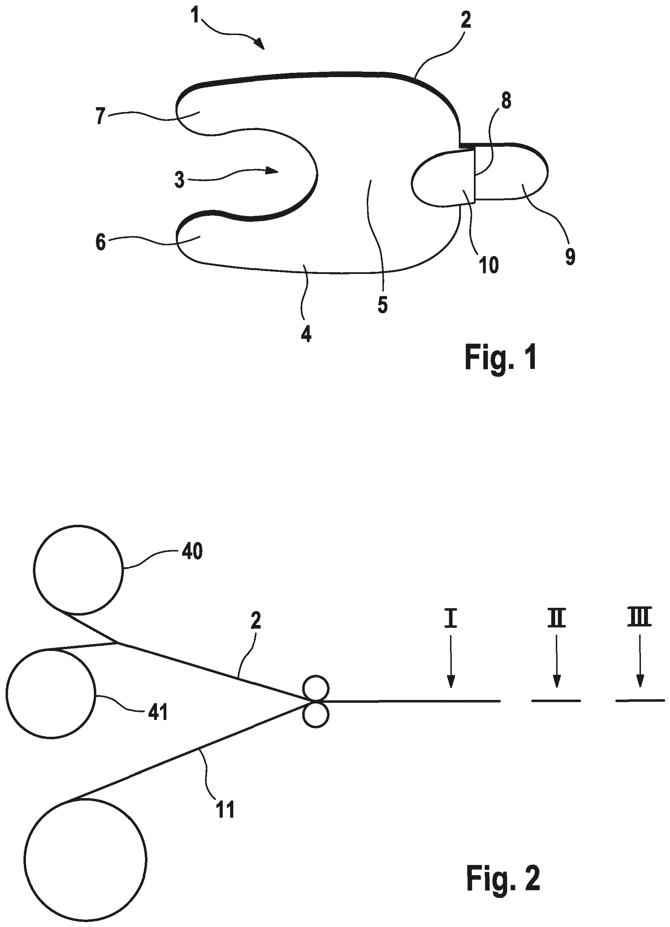

Fig. 1 zeigt ein Ausführungsbeispiel eines medizinischen Sensors 1. Bei diesem Ausführungsbeispiel ist der medizinische Sensor ein elektrischer Feuchtigkeitssensor, der auf die Haut des Patienten an der Punktionsstelle aufgelegt werden kann. Der Feuchtigkeitssensor ist ein Pad, das ein textiles Flächengebilde 2 (Gewebe) aufweist, das aus leitfähigen und nicht leitfähigen Kett- und Schussfäden hergestellt wird. Die leitfähigen Kett- und Schussfäden bilden in dem Gewebe 2 eine elektrisch leitfähige Struktur zur Feuchtigkeitsmessung. An der beim Gebrauch des Sensors dem Patienten zugewandten Seite weist der Sensor 1 eine Klebe- oder Adhäsionsschicht auf, die mit einem abziehbaren Deckmaterial 4 abgedeckt ist.

Der medizinische Sensor 1 weist einen mittleren Bereich 5 mit zwei Schenkeln 6, 7 auf, die einen halbkreisförmigen Ausschnitt 3 seitlich umschließen. An den mittleren Bereich 5 schließt sich eine Anschlusslasche 9 an, die den beiden Schenkeln gegenüberliegt. An der Anschlusslasche 9 befinden sich die in Fig. 1 nicht dargestellten Anschlusskontakte für die elektrisch leitfähige Struktur. Das Deckmaterial 4 ist im Bereich der Anschlusslasche 9 umgelegt und mit einer Falzkante 8 versehen. Der umgelegte Abschnitt des Deckmaterials 4 bildet daher eine Abziehlasche 10.

Fig. 2 zeigt die wesentlichen Verfahrensschritte zur Herstellung des Sensors. Zur Herstellung der Gewebebahn 2 werden die Kettfäden und die Schussfäden 40, 41 zugeführt. Während des Webprozesses wird eine Lage 11 zugeführt, mit der die Gewebebahn 2 kaschiert wird. Nach der Herstellung des Gewebes 2 erfolgen weitere dem Fachmann bekannte Verfahrensschritte, zu denen beispielsweise das Veredeln, insbesondere das Waschen, das Fixieren oder eine Wärmebehandlung zählen. In einem weiteren Verfahrensschritt I werden die einzelnen Sensoren separiert. Die Sensoren werden in einem weiteren Prozessschritt II überprüft. Schließlich werden die Sensoren konfektioniert III.

Die mit der Gewebebahn 2 zu kaschierende Lage 11 ist eine in bestimmten Bereichen doppelseitig klebende oder haftende Folie oder Barriereschicht, die an einer Seite mit einem Deckmaterial 4 (Liner) abgedeckt ist. Die beiden Klebe- oder Adhäsionsschichten der Folie können unterschiedliche Eigenschaften haben. Die Klebe- oder Adhäsionsschicht an der einen Seite ist dafür bestimmt, die Gewebebahn 2 und die Folie der Lage 11 fest miteinander zu verbinden, während die Klebe- oder Adhäsionsschicht an der anderen Seite nur dem Haften des abziehbaren Deckmaterials 4 und der Adhäsion des Sensors auf der Haut des Patienten dient. Das Deckmaterial 4 dient dem Schutz des Sensors 1 und wird vor dem Aufbringen des Sensors auf die Haut des Patienten abgezogen und verworfen (Fig. 1).

An der dem Deckmaterial zugewandten Seite weist die Folie der Lage 11 in Längsrichtung (Produktionsrichtung) verlaufende streifenförmige Bereiche auf, die frei von der Klebe- oder Adhäsionsschicht sind, so dass das Deckmaterial 4 in diesen Bereichen nicht an der Folie haftet und bei den separierten Sensoren Laschen bilden (Fig. 1).

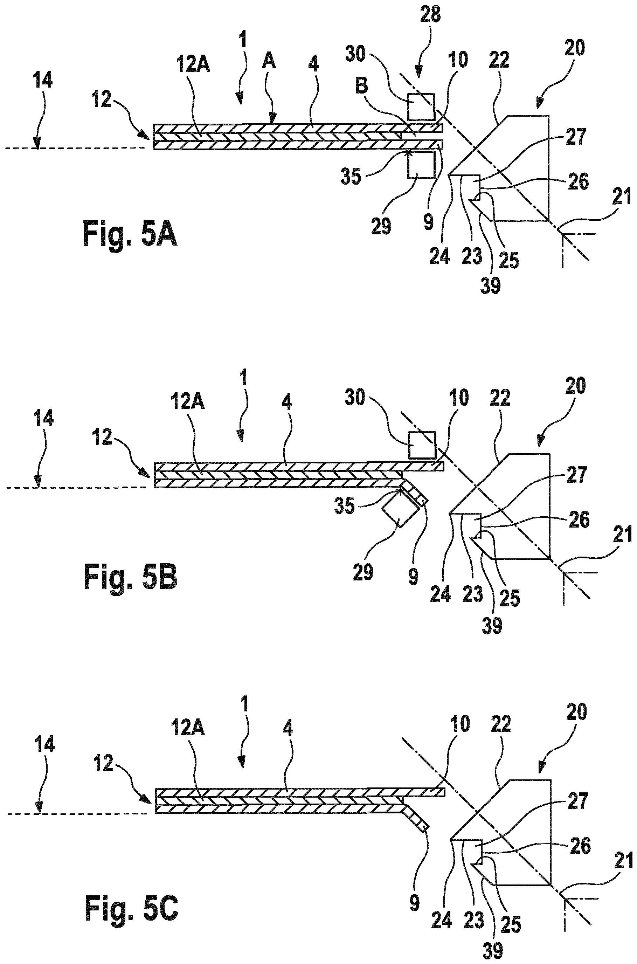

Fig. 5A zeigt einen Schnitt durch den separierten Sensor 1, der in die nur stark vereinfacht schematisch dargestellte erfindungsgemäße Vorrichtung eingelegt ist. Die Gewebebahn 2 und die Folie oder Barriereschicht der Lage 11 werden nachfolgend als Trägermaterial 12 des Sensors 1 bezeichnet. In Fig. 5A ist das Trägermaterial 12 als eine zusammenhängende Lage dargestellt, da für die Erfindung unerheblich ist, ob die Trägerschicht aus mehreren Schichten oder Lagen besteht. Für die Erfindung ist nur von Bedeutung, dass die Trägerschicht in einem Bereich zur Bildung der Lasche frei von der Klebe- oder Adhäsionsschicht ist.

Das Trägermaterial 12 weist einen ersten Abschnitt A, der mit der Adhäsions- oder Klebeschicht 12A versehen ist, und einen zweiten Abschnitt B auf, in dem das Trägermaterial 12 nicht mit einer Adhäsions- oder Klebeschicht versehen ist. Bei dem vorliegenden Ausführungsbeispiel ist der von der Adhäsions- oder Klebeschicht freie Abschnitt B des Trägermaterials 12 die Anschlusslasche 9. Da das Deckmaterial 4 in dem von der Adhäsions- oder Klebeschicht freien Abschnitt nicht an dem Trägermaterial 12 haftet, bildet das Deckmaterial 4 in diesem Abschnitt die Abziehlasche 10. Um die Abziehlasche 10 leicht greifen zu können, wird das Deckmaterial 4 gefalzt, so dass die Lasche von dem Trägermaterial absteht. Da der Sensor 1 im Bereich der Lasche wegen der dort fehlenden Adhäsions- oder Klebeschicht nicht an der Haut des Patienten haftet, kann der Sensor einfach mit einer elektrischen Anschlussklemme kontaktiert werden und nach dem Gebrauch an der Lasche leicht gegriffen und von der Haut abgezogen werden.

Für die Produktion der Sensoren 1 in großen Stückzahlen wird angestrebt, dass sich das Deckmaterial 4 nach dem Separieren der Sensoren einfach falzen lässt. Dabei sollte eine saubere Falzkante 8 an einer definierten Position geschaffen werden.

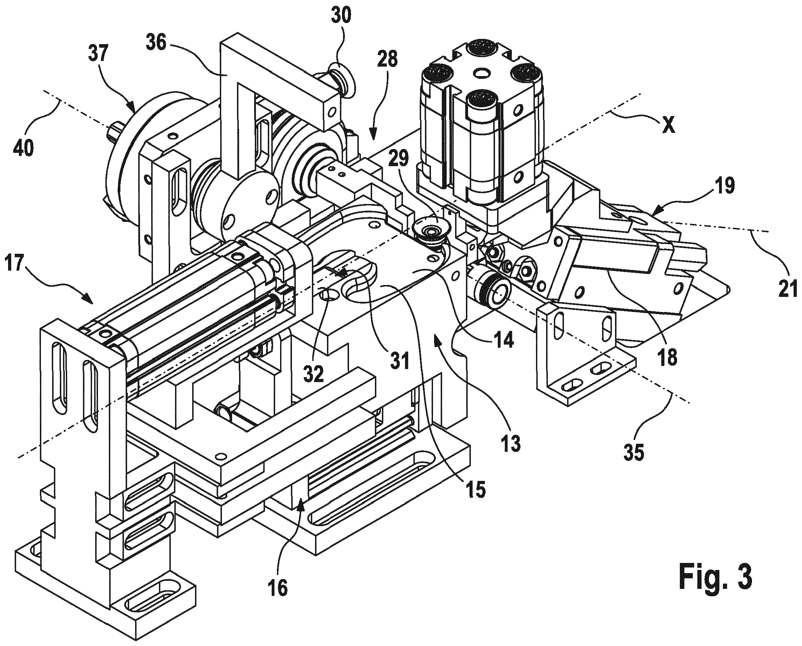

Nachfolgend wird die erfindungsgemäße Vorrichtung zum Falzen des Deckmaterials 4 unter Bezugnahme auf die Figuren 3 und 4 sowie 5A bis 5H im Einzelnen beschrieben.

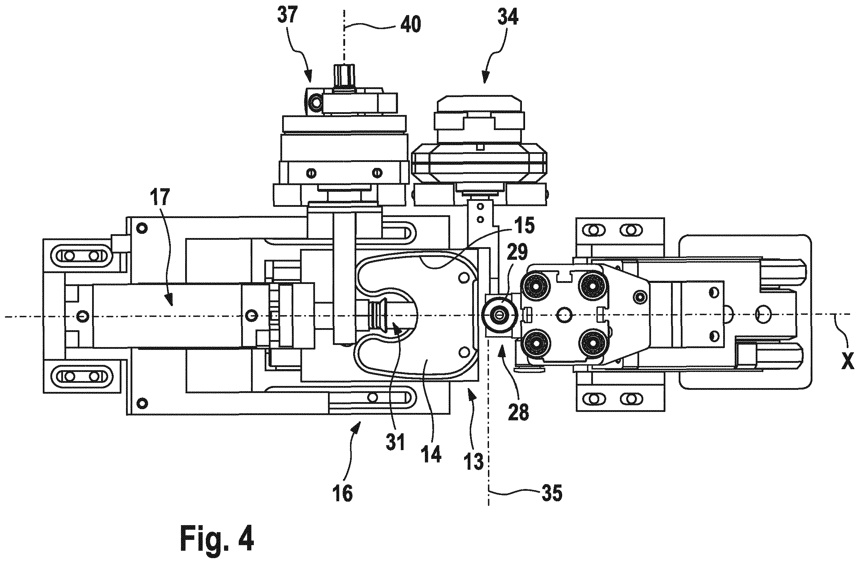

Die Figuren 3 und 4 zeigen die wesentlichen Komponenten der erfindungsgemäßen Vorrichtung, während in den Figuren 5A bis 5H die einzelnen Verfahrensschritte illustriert werden, mit denen das Deckmaterial 4 umgelegt und die Falzkante 8 hergestellt wird. In den Figuren werden für die einander entsprechenden Teile dieselben Bezugszeichen verwendet.

Die erfindungsgemäße Vorrichtung weist eine Halteeinrichtung 13 zur Fixierung des Sensors 1 auf. Die Halteeinrichtung 13 weist eine Auflagefläche 14 mit einer konturierten Vertiefung 15 auf. Die Vertiefung 15 ist derart ausgebildet, dass der mittlere Bereich 5 und die beiden Schenkel 6, 7 des Sensors 1 passend eingelegt werden können. Sie erstreckt sich bis an den Rand der Auflagefläche 14, so dass sich die Anschluss- und Abziehlasche 9, 10 des Sensors über die Auflagefläche 14 nach vorne hinaus erstrecken. Die zentrale Längsachse der Vorrichtung, die durch den auf der Auflagefläche 14 aufliegenden Sensor verläuft, ist mit dem Bezugszeichen X bezeichnet.

Die Halteeinrichtung 13 ist mit einer Linearführung auf der Längsachse X der Vorrichtung um eine vorgegebene Wegstrecke zwischen einer vorderen und einer hinteren Position verschiebbar geführt. Der Antrieb der längsverschiebbar geführten Halteeinrichtung 13 erfolgt bei dem vorliegenden Ausführungsbeispiel mit einer pneumatischen Antriebsvorrichtung. Linearführung und Antriebsvorrichtung bilden eine Einheit 16, die unter der Halteeinrichtung 13 angeordnet ist. Bei dieser Einheit 16 kann es sich um einen pneumatischen Zylinder mit Linearführung handeln, der auch als Minischlitten bezeichnet wird.

Vor der Halteeinrichtung 13 befinden sich eine Linearführung und Antriebsvorrichtung für ein in den Figuren 3 und 4 nicht erkennbares Werkzeug zum Falzen. Linearführung und Antriebsvorrichtung bilden wieder eine Einheit, die mit der Bezugsziffer 18 bezeichnet ist. Das Werkzeug kann entlang einer Achse 21 bewegt werden, die schräg zu der Auflagefläche 14 verläuft. Der Winkel zwischen der Achse 21 und der Auflagefläche 14 liegt vorzugsweise zwischen 30° und 60°, insbesondere 45°.

Die Figuren 5A bis 5H zeigen den Sensor 1 und das Werkzeug 20 der erfindungsgemäßen Vorrichtung in stark vereinfachter Darstellung, wobei der Sensor 1 auf der Auflagefläche 14 der nicht dargestellten Halteeinrichtung aufliegt. Der vordere Abschnitt des Trägermaterials 12 bildet die Anschlusslasche 9 mit den elektrischen Anschlusskontakten und der vordere Abschnitt des Deckmaterials 4, das nicht an dem Trägermaterial haftet, bildet die Abziehlasche 10.

In den Figuren 5A bis 5H ist der Vorschub des Werkzeugs 20 zum Falzen auf der schräg zu der Oberfläche des Sensors verlaufenden Achse 21 von der Ausgangsposition in die Endposition in einzelnen Schritten dargestellt.

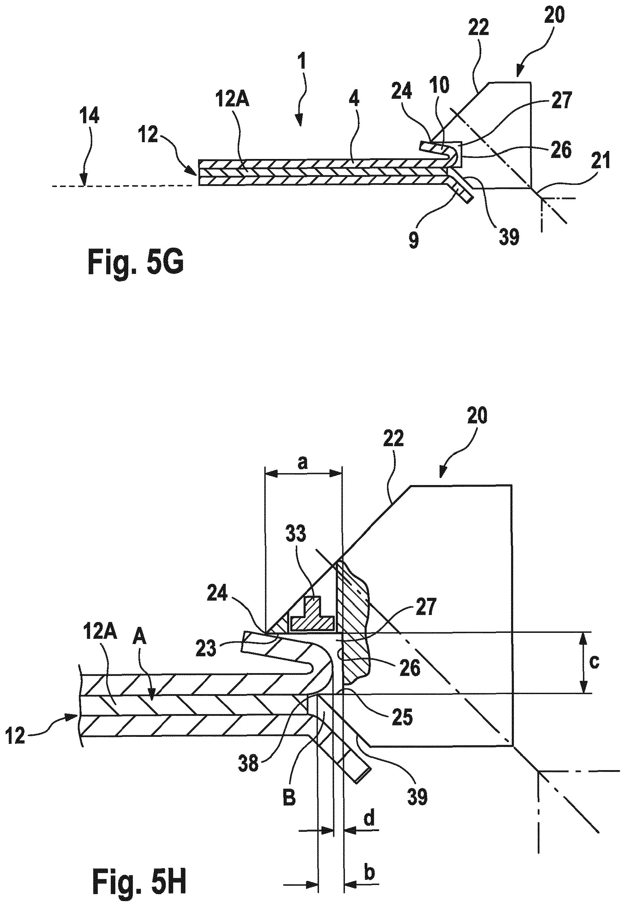

Das Werkzeug 20 weist eine erste Anlagefläche 22 auf, die orthogonal auf der Achse 21 steht, auf der sich das Werkzeug bewegt (Fig. 5A, Fig. 5H). In der Ausgangsposition des Werkzeugs 20 ist die erste Anlagefläche 22 der Abziehlasche 10 zugewandt. An die erste Anlagefläche 22 schließt sich eine zweite Anlagefläche 23 an, die parallel zu der Sensoroberfläche verläuft. Die erste und zweite Anlagefläche 22, 23 schneiden sich in einer Kante 24, die in der Ausgangsposition des Werkzeugs der Abziehlasche 10 zugewandt ist. Der zweiten Anlagefläche 23 liegt eine parallel zu der Sensoroberfläche verlaufende dritte Anlagefläche 25 im Abstand gegenüber. Die zweite und dritte Anlagefläche 23, 25 verbindet eine vierte Anlagefläche 26, die senkrecht auf der Sensoroberfläche steht. Die zweite und dritte Anlagefläche 23, 25 bilden die seitlichen Begrenzungen einer rechteckförmigen Nut 27 in dem Werkzeug, wobei die vierte Anlagefläche der Nutgrund 26 ist.

Die zweite Anlagefläche 23 hat eine Länge a, die größer ist als die Länge b der dritten Anlagefläche 25. Die Länge c der vierten Anlagefläche 26 ist größer als die Länge b der dritten Anlagefläche 25 und kleiner als die Länge a der zweiten Anlagefläche 23.

An die vierte Anlagefläche schließt sich eine zurückspringende Schrägfläche 39 an, die vorzugsweise parallel zu der Achse 21 verläuft, auf der sich das Werkzeug 20 bewegt.

Zum Trennen des von der Adhäsions- oder Klebeschicht 12A freien Abschnitts A des Trägermaterials 12, d.h. der Anschlusslasche 9, von der Abziehlasche 10 weist die erfindungsgemäße Vorrichtung eine Einrichtung 28 auf, die über ein unteres Greifelement 29 und ein oberes Haltelement 30 verfügt. Das Greifelement 29 und das Halteelement 30 sind als vorzugsweise tellerförmige Saugnäpfe ausgebildet, die an eine nicht dargestellte Vorrichtung zur Erzeugung eines Unterdrucks angeschlossen sind, so dass Abzieh- und Anschlusslasche 9, 10 angesaugt werden können.

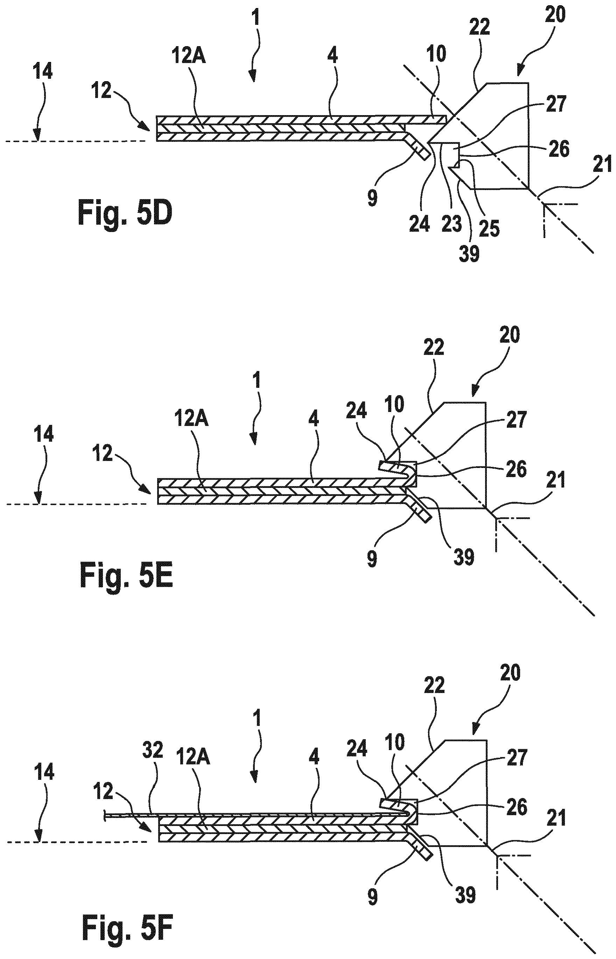

Darüber hinaus verfügt die erfindungsgemäße Vorrichtung über eine Positionierungseinrichtung 31, die ein Positionierungselement 32 aufweist, das mit einer vorzugsweisen pneumatischen Antriebsvorrichtung 17 auf einer parallel zu der Auflagefläche 14 verlaufenden Achse in Längsrichtung X der Vorrichtung über eine kurze Wegstrecke, beispielsweise 2 mm, zwischen einer zurückgezogenen Position und einer vorgeschobenen Position, bewegbar ist (Fig. 5F). Das Positionierungselement 32 ist eine flache Zunge, die in Längsrichtung über die Oberfläche des auf der Auflagefläche 14 aufliegenden Sensors 1 geleitet (Fig. 5F).

Zur Herstellung der Falzkante 8 weist die erfindungsgemäße Vorrichtung ein langgestrecktes Andruckelement 33 auf, das sich quer zur Längsrichtung X der Vorrichtung erstreckt. Das Andruckelement 33 ist in dem Werkzeug 20 zwischen einer angehobenen Position und einer abgesenkten Position auf einer Achse bewegbar, die orthogonal auf der Auflagefläche 14 steht. Das in dem Werkzeug verschiebbar geführte Andruckelement 33 ist in Fig. 5H schematisch dargestellt.

Das Greifelement 29 ist mit einer vorzugsweisen pneumatischen Antriebseinheit 34 um eine Achse 35 schwenkbar, die quer zur Längsrichtung der Vorrichtung und parallel zu der Auflagefläche 14 verläuft, insbesondere in der Ebene der Auflagefläche liegt, so dass sich das Greifelement 29 aus einer horizontalen Position, in der das Greifelement an der Unterseite der Anschlusslasche 9 anliegt, in eine schräggestellte Position bewegt, in der die Anschlusslasche 9 nach unten umgebogen und somit von der Abziehlasche 10 getrennt ist (Fig. 5A und 5B).

Das Haltelement 30 ist an dem freien Ende eines Schwenkarms 36 befestigt, der mit einer vorzugsweisen pneumatischen Antriebseinheit 37 um eine Achse 40 schwenkbar ist, die quer zur Längsrichtung X der Vorrichtung und parallel zu der Auflagefläche 14 verläuft, so dass sich das Haltelement 30 auf einer Kreisbahn zwischen einer zurückgezogenen Position und einer Position bewegt, in der das Halteelement an der Oberseite der Abziehlasche 10 anliegt.

Die einzelnen Komponenten der erfindungsgemäßen Vorrichtung werden von der nicht dargestellten Steuereinheit derart angesteuert, dass die nachfolgenden Verfahrensschritte durchgeführt werden.

Zunächst werden das Haltelement 30 und das Greifelement 29 in die Position bewegt, in der das Halteelement 30 an der Oberseite der Abziehlasche 10 und das Greifelement 29 an der Unterseite der Anschlusslasche 9 anliegen und Abzieh- und Anschlusslasche ansaugen (Fig. 5A). Dann wird das Greifelement 29 um die Achse 35 gedreht, so dass die Anschlusslasche 9 von der Abziehlasche 10 getrennt wird (Fig. 5B). Dieser Vorgang wird vorzugsweise zur Vermeidung von Fehlfunktionen einmal wiederholt, so dass die Anschlusslasche zweimal umgelegt wird. Das Greifelement 29 verbleibt nun in der schräggestellten Position, während das Haltelement 30 nach dem Abschalten des Unterdrucks in die Ausgangsposition zurückgefahren wird. Das Halteelement 30 hält die Anschlusslasche 9 in der nach unten abgewinkelten Position, so dass die Lasche bei den nachfolgenden Schritten nicht beschädigt werden kann.

Fig. 5C zeigt das Werkzeug 20 zum Falzen in der Ausgangsposition, wobei das Halteelement 30 zurückgezogen und das Greifelement nicht dargestellt ist. Das Werkzeug 20 wird von der Ausgangsposition in die Endposition (Fig. 5E) verfahren, wobei sich die Kante 24 des Werkzeugs 20 an der vorderen Kante der Folie 11 vorbei bewegt (Fig. 5D), d.h. von unten auf den Sensor 1 an der Grenze zwischen dem von der Adhäsions- oder Klebeschicht freien und dem mit der Adhäsions- oder Klebeschicht versehenen Abschnitt trifft. Während des Vorschubs des Werkzeugs 20 wird die Abziehlasche 10 an der Unterseite von der ersten Anlagefläche 22 des Werkzeugs 20 erfasst und nach oben gebogen, wobei die Abziehlasche 10 an der ersten Anlagefläche entlanggleitet (Fig. 5D). Die Abziehlasche 10 legt sich nunmehr um, bis die Lasche in der Nut 27 zu liegen kommt. Dabei trifft die vordere Kante 38, an der sich die dritte Anlagefläche 25 und die Schrägfläche 39 schneiden, auf die vordere Kante der Folie, d.h. von unten auf den Sensor 1 an der Grenze zwischen dem von der Adhäsions- oder Klebeschicht freien und dem mit der Adhäsions- oder Klebeschicht versehenen Abschnitt des Sensors. Die Anschlusslasche 10 legt sich an die Schrägfläche 39 des Werkzeugs 20 an (Fig. 5E).

Anschließend wird das Positionierungselement 32 aus der zurückgezogenen Position in die vorgeschobene Position verfahren, so dass das Positionierungselement 32 den umgebogenen Abschnitt des Deckmaterials 4 gegen den Nutgrund 26 drückt (Fig. 5F). Danach wird das Positionierungselement 32 wieder zurückgezogen.

Darauf hin wird die Halteinrichtung 13 um eine vorgegebene Wegstrecke d zurückgefahren, wobei der umgebogene Abschnitt des Deckmaterials 4 um die Strecke d zurückgezogen wird, so dass der umgebogene Abschnitt zur Herstellung der Falzkante 8 exakt in der Nut 27 positioniert ist (Fig. 5G, Fig. 5H). Diese Strecke ist sehr klein und entspricht dem sogenannten Spaltmaß des Werkzeugs.

Nunmehr wird das Andruckelement 33 aus der angehobenen Position in die abgesenkte Position bewegt, so dass das Andruckelement 33 zur Herstellung einer definierten Falzkante 8 (Fig. 1) von oben auf die umgelegte Abziehlasche 10 drückt (Fig. 5H). Das Andruckelement 33 wird dann wieder in die angehobene Ausgangsposition zurückgefahren. Danach werden wieder das Werkzeug 20 in seine Ausgangsposition, die Halteeinrichtung 13 in die vorgeschobene Position und das Halteelement 30 in seine Grundstellung verfahren.

Die Beschickung der erfindungsgemäßen Vorrichtung kann mit einer nicht dargestellten Beschickungseinrichtung vollautomatisch erfolgen.

## Patentansprüche

1. Verfahren zum Falzen eines Deckmaterials an einem medizinischen Sensor mit einem Trägermaterial, das an einer Seite in einem ersten Abschnitt eine Adhäsions- oder Klebeschicht und in einem zweiten Abschnitt keine Adhäsions- oder Klebeschicht aufweist, wobei auf das Trägermaterial ein Deckmaterial aufgebracht ist, das in einem Abschnitt, der dem zweiten Abschnitt des Trägermaterials gegenüberliegt, eine Lasche bildet,
mit folgenden Verfahrensschritten:
Anordnen des Verbundes von Trägermaterial und Deckmaterial in einer Ebene,
Anordnen eines Werkzeugs, das eine der Lasche zugewandte erste Anlagefläche und eine der Lasche abgewandte zweite Anlagefläche aufweist, auf der Seite des Verbundes von Trägermaterial und Deckmaterial, an der sich die Lasche befindet, wobei sich die Anlageflächen des Werkzeugs in einer parallel zu der Ebene des Verbundes von Trägermaterial und Deckmaterial verlaufenden Kante schneiden, die dem Verbund von Trägermaterial und Deckmaterial zugewandt ist,
Bewegen des Werkzeugs auf einer schräg zu der Ebene des Verbundes von Trägermaterial und Deckmaterial verlaufenden Achse, so dass die Lasche von dem Werkzeug erfasst und mit dem Werkzeug umgebogen wird und
Ausüben von Druck auf den gebogenen Abschnitt des umgelegten Deckmaterials zur Herstellung einer Falzkante.

2. Verfahren nach Anspruch 1, **dadurch gekennzeichnet, dass** der von der Adhäsions- oder Klebeschicht freie Abschnitt des Trägermaterials von der Lasche getrennt wird, bevor das Werkzeug in Bewegung gesetzt wird.

3. Verfahren nach Anspruch 1 oder 2, **dadurch gekennzeichnet, dass** das Werkzeug eine dritte Anlagefläche aufweist, die der zweiten Anlagefläche im Abstand gegenüberliegt, und dass die zweite und dritte Anlagefläche die seitlichen Begrenzungen einer Nut in dem Werkzeug bilden, wobei der umgebogene Abschnitt des Deckmaterials gegen den Nutgrund gedrückt wird, bevor der umgebogene Abschnitt gegen die dritte Anlagefläche gedrückt wird.

4. Verfahren nach einem der Ansprüche 1 bis 3, **dadurch gekennzeichnet, dass** der Verbund von Trägermaterial und Deckmaterial auf einer Auflagefläche derart angeordnet wird, dass sich die Lasche über die Auflagefläche hinaus erstreckt.

5. Verfahren nach einem der Ansprüche 1 bis 4, **dadurch gekennzeichnet, dass** zum Trennen des von der Adhäsions- oder Klebeschicht freien Abschnitts des Trägermaterials von der Lasche ein Greifelement, das an dem Trägermaterial an der dem Deckmaterial abgewandten Seite angreift, um eine parallel zu der Ebene des Verbundes von Trägermaterials und Deckmaterial verlaufende Achse verschwenkt wird.

6. Vorrichtung zum Falzen eines Deckmaterials (4) an einem medizinischen Sensor (1) mit einem Trägermaterial (12), das an einer Seite in einem ersten Abschnitt (A) eine Adhäsions- oder Klebeschicht (12A) und in einem zweiten Abschnitt (B) keine Adhäsions- oder Klebeschicht aufweist, wobei auf das Trägermaterial (12) ein Deckmaterial (4) aufgebracht ist, das in einem Abschnitt, der dem zweiten Abschnitt des Trägermaterials gegenüberliegt, eine Lasche (10) bildet, wobei die Vorrichtung zum Falzen aufweist:
eine Halteeinrichtung (13), die eine Auflagefläche (14) zum Auflegen des Sensors aufweist,
ein neben der Halteeinrichtung (13) angeordnetes Werkzeug (20), das eine erste Anlagefläche (22) und eine zweite Anlagefläche (23) aufweist, die sich in einer parallel zu der Auflagefläche (14) verlaufenden Kante (24) schneiden, wobei das Werkzeug (20) auf einer schräg zu der Ebene der Auflagefläche (14) verlaufenden Achse (21) bewegbar geführt ist, und
ein bewegbar geführtes Andruckelement (33) zum Ausüben von Druck zur Herstellung einer Falzkante.

7. Vorrichtung nach Anspruch 6, **dadurch gekennzeichnet, dass** die erste Anlagefläche (22) im Wesentlichen orthogonal zu der Achse (21) verläuft, auf der das Werkzeug (20) bewegbar geführt ist.

8. Vorrichtung nach Anspruch 6 oder 7, **dadurch gekennzeichnet, dass** die zweite Anlagefläche (23) in einer Ebene liegt, die im Wesentlichen parallel zu der Auflagefläche (14) verläuft.

9. Vorrichtung nach einem der Ansprüche 6 bis 8, **dadurch gekennzeichnet, dass** das Andruckelement (33) in dem Werkzeug (20) auf einer Achse bewegbar geführt ist, die orthogonal zu der Auflagefläche (14) verläuft.

10. Vorrichtung nach einem der Ansprüche 6 bis 9, **dadurch gekennzeichnet, dass** das Werkzeug (20) eine dritte Anlagefläche (25) aufweist, die der zweiten Anlagefläche (23) im Abstand gegenüberliegt, wobei die zweite und dritte Anlagefläche (22, 25) die seitlichen Begrenzungen einer Nut (27) in dem Werkzeug (20) bilden.

11. Vorrichtung nach einem der Ansprüche 6 oder 10, **dadurch gekennzeichnet, dass** ein Positionierungselement (32) vorgesehen ist, das auf einer parallel zu der Auflagefläche (14) verlaufenden Achse verschiebbar geführt ist.

12. Vorrichtung nach einem der Ansprüche 6 bis 11, **dadurch gekennzeichnet, dass** die Halteeinrichtung (13) auf einer parallel zu der Auflagefläche (14) verlaufenden Achse verschiebbar geführt ist.

13. Vorrichtung nach einem der Ansprüche 6 bis 12, **dadurch gekennzeichnet, dass** die Auflagefläche (14) eine konturierte Vertiefung (15) zum Einlegen des zweiten Abschnitts des Trägermaterials (12) und des auf den zweiten Abschnitt des Trägermaterials (12) aufgebrachten Deckmaterials (4) aufweist, wobei sich die konturierte Vertiefung (15) derart bis an den Rand der Auflagefläche (14) erstreckt, dass sich die Lasche (10) des in die Vertiefung eingelegten Sensors (1) über die Auflagefläche (14) hinaus erstreckt.

14. Vorrichtung nach einem der Ansprüche 6 bis 13, **dadurch gekennzeichnet, dass** ein Greifelement (29) zum Trennen des von der Adhäsions- oder Klebeschicht freien Abschnitts des Trägermaterials (12) von der Lasche (10) neben der Halteeinrichtung (13) unterhalb der Auflagefläche (14) angeordnet ist, wobei das Greifelement (29) um eine parallel zu der Auflagefläche (14) verlaufenden Achse verschwenkbar ist.

15. Vorrichtung nach einem der Ansprüche 6 bis 14, **dadurch gekennzeichnet, dass** ein Halteelement (30) zum Halten der Lasche (10) neben der Halteeinrichtung (13) oberhalb der Auflagefläche (14) angeordnet ist.

## Claims

1. A method for folding a cover material on a medical sensor having a carrier material which, on one side, in a first section, has an adhesion or adhesive layer and, in a second section, no adhesion or adhesive layer, wherein a cover material, which forms a tab in a section which overlaps the second section of the carrier material, is applied to the carrier material,
having the following method steps:
arrangement of the combination of carrier material and cover material in one plane,
arrangement of a tool which has a first contact surface facing towards the tab and a second contact surface facing away from the tab on the side of the combination of carrier material and cover material on which the tab is located, wherein the contact surfaces of the tool intersect at an edge which runs parallel to the plane of the combination of carrier material and cover material and faces the combination of carrier material and cover material,
movement of the tool on an axis which runs at an angle to the plane of the combination of carrier material and cover material, so that the tab is gripped by the tool and folded over by the tool, and
exertion of pressure on the folded section of the folded-over cover material to produce a folded edge.

2. The method as claimed in claim 1, **characterized in that** the section of the carrier material which is free from the adhesion or adhesive layer is separated from the tab before the tool is set in motion.

3. The method as claimed in claim 1 or 2, **characterized in that** the tool has a third contact surface which lies opposite and at a distance from the second contact surface, and that the second and third contact surface form the lateral boundaries of a groove in the tool, wherein the folded-over section of the cover material is pressed against the base of the groove before the folded-over section is pressed against the third contact surface.

4. The method as claimed in one of claims 1 to 3, **characterized in that** the combination of carrier material and cover material is arranged on a contact surface in such a way that the tab extends beyond the contact surface.

5. The method as claimed in one of claims 1 to 4, **characterized in that** a gripper element, which grips the side of the carrier material facing away from the cover material, is swiveled about an axis which runs parallel to the plane of the combination of carrier material and cover material in order to separate the section of the carrier material which is free from the adhesion or adhesive layer from the tab.

6. A device for folding a cover material (4) on a medical sensor (1) having a carrier material (12) which, on one side, in a first section (A), has an adhesion or adhesive layer (12A) and, in a second section (B), no adhesion or adhesive layer, wherein a cover material (4), which forms a tab (10) in a section which overlaps the second section of the carrier material, is applied to the carrier material (12),
wherein the folding device has:
a holding device (13), which has a contact surface (14) on which to place the sensor,
a tool (20) which is arranged next to the holding device (13) and has a first contact surface (22) and a second contact surface (23) which intersect at an edge (24) which runs parallel to the contact surface (14), wherein the tool (20) is movably guided on an axis (21) which runs at an angle to the plane of the contact surface (14), and
a movably guided contact-force element (33) for exerting pressure to produce a folded edge.

7. The device as claimed in claim 6, **characterized in that** the first contact surface (22) runs substantially orthogonally with respect to the axis (21) on which the tool (20) is movably guided.

8. The device as claimed in claim 6 or 7, **characterized in that** the second contact surface (23) lies in a plane which runs substantially parallel to the contact surface (14).

9. The device as claimed in one of claims 6 to 8, **characterized in that** the contact-force element (33) is movably guided in the tool (20) on an axis which runs orthogonally with respect to the contact surface (14).

10. The device as claimed in one of claims 6 to 9, **characterized in that** the tool (20) has a third contact surface (25) which lies opposite and at a distance from the second contact surface (23), the second and third contact surface (22, 25) forming the lateral boundaries of a groove (27) in the tool (20).

11. The device as claimed in one of claims 6 to 10, **characterized in that** a positioning element, which is movably guided on an axis which runs parallel to the contact surface (14), is provided.

12. The device as claimed in one of claims 6 to 11, **characterized in that** the holding device (13) is movably guided on an axis which runs parallel to the contact surface (14).

13. The device as claimed in one of claims 6 to 12, **characterized in that** the contact surface (14) has a contoured depression (15) in which to place the second section of the carrier material (12) and the cover material (4) which is applied to the second section of the carrier material (12), wherein the contoured depression (15) extends as far as the edge of the contact surface (14) in such a way that the tab (10) of the sensor (1) which is placed in the depression extends beyond the contact surface (14).

14. The device as claimed in one of claims 6 to 13, **characterized in that** a gripper element (29) for separating the section of the carrier material (12) which is free from the adhesion or adhesive layer from the tab (10) is arranged next to the holding device (13) below the contact surface (14), wherein the gripper element (29) can be swiveled about an axis which runs parallel to the contact surface (14).

15. The device as claimed in one of claims 6 to 14, **characterized in that** a holding element (30) for holding the tab (10) is arranged next to the holding device (13) above the contact surface (14).

## Revendications

1. Procédé servant à plier un matériau de recouvrement au niveau d'un dispositif de détection médical, avec un matériau de support qui présente d'un côté, dans un premier segment, une couche d'adhérence ou de collage et dans un deuxième segment, ne présente pas de couche d'adhérence ou de collage, dans lequel sur le matériau de support est appliqué un matériau de recouvrement qui forme un rabat dans un segment qui est opposé au deuxième segment du matériau de support,
avec les étapes de procédé suivantes :
la disposition du composite du matériau de support et du matériau de recouvrement dans un plan,
la disposition d'un outil qui présente une première surface d'appui tournée vers le rabat et une deuxième surface d'appui détournée du rabat sur le côté du composite du matériau de support et du matériau de recouvrement sur lequel se trouve le rabat, dans lequel les surfaces d'appui de l'outil se coupent en un bord évoluant parallèlement au plan du composite du matériau de support et du matériau de recouvrement, qui est tourné vers le composite du matériau de support et du matériau de recouvrement,
le déplacement de l'outil sur un axe évoluant perpendiculairement au plan du composite du matériau de support et du matériau de recouvrement, de sorte que le rabat soit saisi par l'outil et soit replié avec l'outil et
l'exercice d'une pression sur le segment plié du matériau de recouvrement retourné pour produire un rebord de pliage.

2. Procédé selon la revendication 1, **caractérisé en ce que** le segment libre de la couche d'adhérence ou de collage du matériau de support est séparé du rabat avant que l'outil soit déplacé.

3. Procédé selon la revendication 1 ou 2, **caractérisé en ce que** l'outil présente une troisième surface d'appui qui est opposée, à distance de la deuxième surface d'appui, et **en ce que** les deuxième et troisième surfaces d'appui forment les limites latérales d'une rainure dans l'outil, dans lequel le segment replié du matériau de recouvrement est appuyé contre le fond de la rainure, avant que le segment replié soit appuyé contre la troisième surface d'appui.

4. Procédé selon l'une des revendications 1 à 3, **caractérisé en ce que** le composite du matériau de support et du matériau de recouvrement est disposé sur une surface d'appui de telle sorte que le rabat s'étende au-delà de la surface d'appui.

5. Procédé selon l'une des revendications 1 à 4, **caractérisé en ce que**, pour la séparation du segment libre de la couche d'adhérence ou de la couche de collage du matériau de support du rabat, un élément de préhension qui est en prise sur le matériau de support sur le côté détourné du matériau de recouvrement, est basculé d'un axe évoluant parallèlement au plan du composite du matériau de support et du matériau de recouvrement.

6. Procédé servant à plier un matériau de recouvrement (4) au niveau d'un dispositif de détection (1) médical, avec un matériau de support (12) qui présente d'un côté, dans un premier segment (A), une couche d'adhérence ou de collage (12A) et dans un deuxième segment (B), ne présente pas de couche d'adhérence ou de collage, dans lequel sur le matériau de support (12) est appliqué un matériau de recouvrement (4) qui forme un rabat (10) dans un segment qui est opposé au deuxième segment du matériau de support,
dans lequel le dispositif de pliage présente :
un système de retenue (13) qui présente une surface d'appui (14) pour placer le dispositif de détection,
un outil (20) disposé à côté du système de retenue (13) qui présente une première surface d'appui (22) et une deuxième surface d'appui (23) qui se coupent en un bord (24) évoluant parallèlement à la surface d'appui (14), dans lequel l'outil (20) est conduit de façon mobile sur un axe (21) évoluant perpendiculairement au plan de la surface d'appui (14), et
un élément de compression (33) conduit de façon mobile pour exercer une pression pour la production d'un rebord de pliage.

7. Dispositif selon la revendication 6, **caractérisé en ce que** la première surface d'appui (22) évolue essentiellement sur un plan orthogonal par rapport à l'axe (21) sur lequel l'outil (20) est conduit de façon mobile.

8. Dispositif selon la revendication 6 ou 7, **caractérisé en ce que** la deuxième surface d'appui (23) se trouve dans un plan qui évolue essentiellement parallèlement à la surface d'appui (14).

9. Dispositif selon l'une des revendications 6 à 8, **caractérisé en ce que** l'élément de compression (33) est conduit de façon mobile dans l'outil (20) sur un axe qui évolue sur le plan orthogonal par rapport à la surface d'appui (14).

10. Dispositif selon l'une des revendications 6 à 9, **caractérisé en ce que** l'outil (20) présente une troisième surface d'appui (25) qui est opposée, à distance de la deuxième surface d'appui (23), dans lequel les deuxième et troisième surfaces d'appui (22, 25) forment les limites latérales d'une rainure (27) dans l'outil (20).

11. Dispositif selon l'une des revendications 6 ou 10, **caractérisé en ce qu'**un élément de positionnement (32) est prévu, qui est conduit de façon à pouvoir coulisser sur un axe évoluant parallèlement à la surface d'appui (14).

12. Dispositif selon l'une des revendications 6 à 11, **caractérisé en ce que** le système de retenue (13) est conduit de façon à pouvoir coulisser sur un axe évoluant parallèlement à la surface d'appui (14).

13. Dispositif selon l'une des revendications 6 à 12, **caractérisé en ce que** la surface d'appui (14) présente une cavité profilée (15) pour placer le deuxième segment du matériau de support (12) et du matériau de recouvrement (4) appliqué sur le deuxième segment du matériau de support (12), dans lequel la cavité profilée (15) s'étend jusqu'à la bordure de la surface d'appui (14) de telle sorte que le rabat (10) du dispositif de détection (1) placé dans la cavité s'étende au-delà de la surface d'appui (14).

14. Dispositif selon l'une des revendications 6 à 13, **caractérisé en ce qu'**un élément de préhension (29) pour séparer le segment du matériau de support (12) sans couche d'adhérence ou de collage, du rabat (10) est disposé à côté du système de retenue (13) au-dessous de la surface d'appui (14), dans lequel l'élément de préhension (29) peut être basculé d'un axe évoluant parallèlement à la surface d'appui (14).

15. Dispositif selon l'une des revendications 6 à 14, **caractérisé en ce qu'**un élément de retenue (30) est disposé pour retenir le rabat (10), à côté du système de retenue (13), au-dessus de la surface d'appui (14).
